Europäisches Patentamt

European Patent Office

Office européen des brevets

(19)

(11) Veröffentlichungsnummer: **0 171 726**
**B1**

(12) **EUROPÄISCHE PATENTSCHRIFT**

(45) Veröffentlichungstag der Patentschrift:
**29.11.89**

(51) Int. Cl.⁴: **A 61 M 11/06**

(21) Anmeldenummer: **85109792.3**

(22) Anmeldetag: **05.08.85**

(54) Inhalator.

(30) Priorität: **09.08.84 DE 3429389**

(73) Patentinhaber: **Brugger, Inge, Prinz-Karl-Strasse 5a, D-8130 Starnberg (DE)**

(43) Veröffentlichungstag der Anmeldung:
**19.02.86 Patentblatt 86/8**

(72) Erfinder: **Die Erfinder haben auf ihre Nennung verzichtet**

(45) Bekanntmachung des Hinweises auf die Patenterteilung:
**29.11.89 Patentblatt 89/48**

(74) Vertreter: **Lehn, Werner, Dipl.-Ing. et al, Hoffmann, Eitle & Partner Patentanwälte Arabellastrasse 4, D-8000 München 81 (DE)**

(84) Benannte Vertragsstaaten:
**AT BE CH DE FR GB IT LI LU NL SE**

(56) Entgegenhaltungen:
**AT-B- 210 560**
**DE-C- 927 920**
**DE-C- 1 147 355**
**US-A- 4 141 369**

ACTORUM AG

## Beschreibung

Die Erfindung betrifft einen Inhalator zum Zerstäuben, Verteilen und Vermischen von flüssigen und pulverförmigen Stoffen in/mit Gas mittels eines Druckgasstromes zur Erzeugung eines Aerosols, welcher einen Zerstäuberkopf mit einem zentralen Düsenkopf und einem Anschlussstutzen für die Druckgasleitung enthält sowie einen Aerosolaustrittsstutzen zum Anschluss an ein Mundstück für den Patienten.

Eine derartige Zerstäubungsvorrichtung ist in der DE-C 927 920 beschrieben. Dabei ist in dem Austrittskegel des Druckgasstromes ein Gasstromsteuer so angeordnet, dass dessen Basiskante den Mantel des Gasaustrittskegels so berühren, dass die Hauptmenge des Gasstromes an den Kanten fächerförmig ausgebreitet wird, wobei die Zuleitungen für das Zerstäubungsgut unterhalb der Luftfächer so angeordnet sind, dass das Zerstäubungsgut in die sich ausbreitenden Fächer eintritt und so eine gute Durchmischung des Zerstäubungsgutes mit dem Druckgas gewährleistet. Da als Druckgas für die Aerosol-Therapie in praktisch allen Fällen Luft zum Einsatz gelangt, erhält man so eine fein verteilte Suspension der flüssigen oder pulverförmigen Wirkstoffe in Luft.

Will man den bekannten Vernebler entweder für einen neuen Patienten oder aber für eine andere Aerosol-Therapie einsetzen, so muss der Vernebler zunächst auseinandergebaut und gründlich gereinigt werden, bevor der Sammelbehälter für die Aufnahme des Wirkstoffes wieder mit einem neuen Wirkstoff gefüllt werden kann. Eine derartige Reinigung ist zeitaufwendig. In vielen Fällen ergibt sich auch die Notwendigkeit einer mehrmaligen Behandlung ein und desselben Patienten, für den dann jeweils wieder ein Vernebler mit dem gleichen Wirkstoff aufgefüllt werden muss.

Eine weitere vorbekannte Zerstäubervorrichtung ist auch in der DE-C 1 147 355 beschrieben.

Es ist daher das Ziel der Erfindung, einen Inhalator der genannten Art zu schaffen, der durch die Trennung der Bauteile für die Handhabung und für den Vernebler eine einfache Austauschbarkeit dieser Bauteile, nämlich von Zerstäuberkopf und Handgriff, ermöglicht. Der einfache Aufbau des Zerstäuberkopfes, seine gedrungene Bauweise sowie seine einfache Zerlegbarkeit zur Reinigung schafft so einen «persönlichen» Vernebler, der ausserhalb des Handgriffs und mit dem Wirkstoff für einen bestimmten Patienten versehen, in einfacher Weise aufbewahrt werden kann und im Fall seiner Wiederverwendung einfach mit dem Handgriff verbindbar ist und so an die Druckgasleitung angeschlossen werden kann.

Durch die getrennte Ausbildung von Handgriff und Zerstäuberkopf lässt sich der Handgriff auch ergonomisch günstiger gestalten als dies bei der bekannten Zerstäubervorrichtung der Fall ist, wo die Gehäusewandung des Verneblers einfach als Handgriff benutzt wurde. Die einfache Austauschbarkeit der Zerstäuberköpfe legt es nahe, denselben Handgriff für mehrere Zerstäuberköpfe zu verwenden, wodurch keine Totzeiten für die Reinigung der Zerstäuberköpfe anfallen, solange ausreichend Zerstäuberköpfe vorrätig sind.

Diese Aufgaben werden mit dem Inhalator der eingangs genannten Art gelöst, der sich dadurch auszeichnet, dass er einen den Zerstäuberkopf umschliessenden zweischaligen Handgriff aufweist, dessen beide Hälften am unteren Ende des Griffteils mittels eines Filmscharniers miteinander verbunden sind und dass der Zerstäuberkopf eine etwa kugelige, den Kugelschalenhälften des Griffteils angepasste Konfiguration aufweist.

Weitere vorteilhafte Ausgestaltungen des erfindungsgemässen Inhalators ergeben sich aus den Unteransprüchen.

Zur Veranschaulichung des neuen Inhalators dient die beigefügte Zeichnung. In der Zeichnung zeigen:

Fig. 1 einen Mittellängsschnitt durch den neuen Inhalator;
Fig. 2 eine perspektivische Ansicht auf die Einzelteile des Inhalators gemäss Fig. 1, wobei der Handgriff in seiner geöffneten Stellung und die wesentlichen Teile des Zerstäuberkopfes auseinander gezogen links vom Handgriff dargestellt sind.

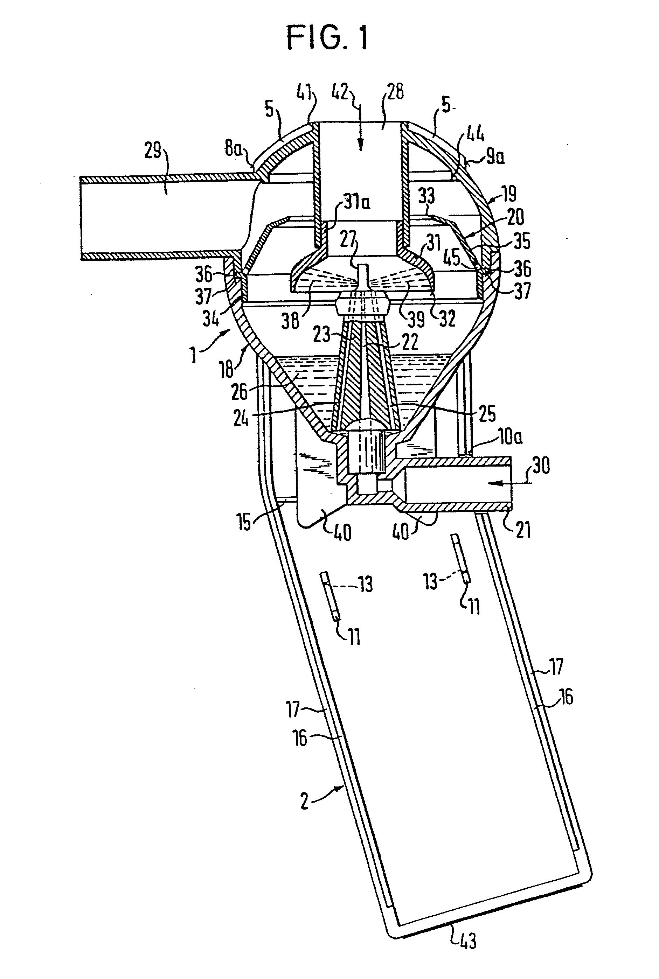

Wie man aus Fig. 1 erkennt, besteht der Inhalator im wesentlichen aus dem Zerstäuberkopf 1, der im oberen Bereich des Handgriffs 2 eingesetzt ist.

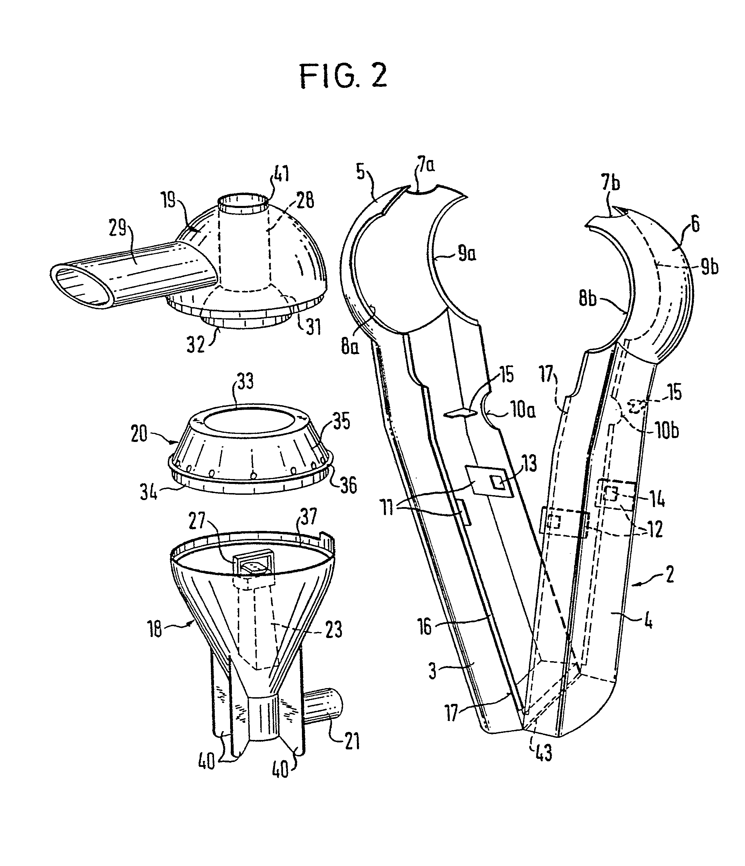

Der Handgriff 2 besteht, wie man am besten aus der rechten Darstellung in Fig. 2 ersieht, aus einer linken Griffschale 3 und einer rechten Griffschale 4, die mittels eines Filmscharniers 43 am unteren Ende des Griffbereichs miteinander verbunden sind. Die in ihrem Querschnitt etwa rechteckigen hohlen Griffschalen 3 und 4 sind in ihrem oberen Bereich zu Kugelschalenhälften 5 und 6 ausgebildet, die sich an den Griffschalenbereich anschliessen. Der Griffschalenbereich kann in einer ergonomisch günstigen Form etwas abgewinkelt sein, um eine ermüdungsfreie Handstellung der rechten Bedienungshand während des Inhalierens für den Benutzer zu gewährleisten. Zu diesem Zweck sind die Griffschalen etwa im Bereich der Öffnungshälften 10a, 10b für den Austritt des Anschlussstutzens 21 für das Druckgas 30 etwas abgewinkelt, wie man aus Fig. 1 erkennt.

Die beiden Öffnungshälften 7a, 7b am Kopf der beiden Kugelschalenhälften 5, 6 dienen zur Aufnahme des zylindrischen Kaminfortsatzes 41 am oberen Ende des koaxialen Zuluftkamins 28, während die Öffnungshälften 8a, 8b den Aerosolaustrittsstutzen 29 der Haube 19 des Zerstäuberkopfes 1 durchlassen und die den Öffnungshälften 8a, 8b gegenüberliegenden in Fig. 2 hinteren Öffnungshälften 9a, 9b, die dem Aerosolaustrittsstutzen 29 gegenüberliegende hintere Kugelkalotte des Zerstäuberkopfes 1 durchtreten lassen. Die Öffnungshälften 10a, 10b umschliessen den Anschlussstutzen 21, der an die Druckgasleitung unter Zwischenschaltung eines einfachen von Hand betätigbaren in der Zeichnung nicht gezeigten Unterbrecherventils anschliessbar ist.

In den Griffschalen befinden sich je zwei Verriegelungslaschen; in der linken Griffschale sind dies die Verriegelungsösen 11 mit entsprechenden Öffnungen 13 zur Aufnahme der Vorsprünge 14, die sich an den Verriegelungshaken 12 in der rechten Griffschale befinden. Die gegenseitig richtige Positionierung der beiden Griffschalen des Handgriffes 2 wird noch durch die Ausbildung von Randleisten 16 an den Rändern der Griffschale 3 und mit diesen zusammenwirkenden entsprechenden Aussparungen (17) an den Rändern der gegenüberliegenden Griffschale (4) begünstigt.

Der Zerstäuberkopf 1 besteht insgesamt aus sechs Bauteilen, von denen in der auseinandergezogenen Darstellung von Fig. 2 links lediglich drei Teile gezeigt sind, nämlich der Behälter 18 mit den vier Positionierungsrippen 40, die gleichzeitig zum Aufstellen für den aus den Griffschalen 3, 4 entnommenen Behälter 18 dienen, der Einsatz 20 und die Haube 19 mit dem Austrittsstutzen 29 für das Aerosol. In die Haube 19 sind der koaxiale Kamin 28 und der Aerosolaustrittsstutzen 29 integriert. Der koaxiale Kamin 28 ist zylindrisch gestaltet, während der Ausstrittsstutzen, wie man aus der perspektivischen Darstellung in Fig. 2 sieht, einen etwa elliptischen Querschnitt aufweist. Der Prallschirm 31 ist in Fig. 2 im zylindrischen Kamin 28 eingesetzt. Der Zuluftkamin 28 kann auch mit dem Prallschirm 31 einteilig ausgebildet sein; die Zweiteiligkeit erleichtert jedoch die Reinigung nach Gebrauch.

Der Behälterteil besteht aus drei Teilen: aus dem Behälter 18 selbst, dem in den Behälter eingesetzten Düsenkopf 23, der sich mit seinem zylindrischen Fussteil in das untere Ende des Behälters 18 einsetzen lässt sowie dem Gasstromsteuer 27, welches auf den Düsenkopf 23 aufgesetzt ist. Die drei in Fig. 2 links auseinandergezogen dargestellten Teile lassen sich axial zusammenschieben und so aufeinandersetzen. Dabei gelangt der Einsatz 20 mit seinem zylindrischen Randflansch 34 in den Behälter 18, wo er mit dem Flansch 36 auf der Ringschulter 37 am oberen Rand des Behälters 18 aufliegt. Anschliessend wird die Haube 19 auf den Behälter 18 mit Einsatz 20 aufgesetzt, wodurch die in Fig. 1 gezeigte kugelige, etwa eiförmige Konfiguration entsteht. Der so gebildete Zerstäuberkopf bildet ein autonomes Bauteil, welches auf den Rippen 40 als Füsse (ohne Handgriff 2) abgestellt werden kann.

Die kurze gedrungene Bauform des Zerstäuberkopfes 1 wird dadurch erreicht, dass man die Bauhöhe des koaxialen Zuluftkamins 28 durch Anordnung von Schikanen für das Aerosol zwischen dem Ort seiner Entstehung und dem Aerosolaustrittsstutzen verkürzt. Bei diesen Schikanen handelt es sich zunächst um den Prallschirm 31 am unteren Ende des Kamins 28. Der Prallschirm 31 ist mittels des zylindrischen Fortsatzes 31a in den Zuluftkamin 28 von unten eingeschoben. Er kann wahlweise auch einteilig mit dem Zuluftkamin ausgebildet sein. Die an den Prallflächen des Gasstromsteuers 27 gebildeten Aerosolfächer 38 und 39 gelangen in Wechselwirkung mit der Innenwandung des glockenförmigen Prallschirms 31, wobei grössere Aerosolteilchen an dieser Wandung nochmals zerkleinert und in den gewünschten Bereich der Teilchengrösse von etwa 0,5 bis 5 μ übergeführt werden. Grössere Tröpfchen scheiden sich am Rand 32 des Prallschirmes 31 ab. Sie tropfen vom Rand 32 in das Zerstäubungsgut 26 im Behälter 18 zurück. Auf seinem weiteren Weg muss der Aerosolstrom um den Rand 32 zwischen Zuluftkamin 28 und Einsatz 20 hindurchstreichen und dabei an einer zweiten Kante, nämlich dem Rand 33 des Einsatzes 20 vorbeistreichen, wo weitere zu grosse Tröpfchen abgeschieden werden, die an der Innenwandung des Wandteils 35 hinablaufen und so zurück zum Zerstäubungsgut 26 am Boden des Behälters 18 gelangen.

Auf diese Weise ist es möglich, ein lungengängiges Aerosol bei relativ kleiner Bauhöhe des Zerstäubers 1 zu erzeugen, das zu einem grossen Anteil in der gewünschten Teilchengrösse vorliegt, wenn es aus dem Austrittsstutzen 29 austritt und dem Patienten zugeführt wird.

Die Wirkungsweise für die Erzeugung des Aerosols ist bekannt. Im Zusammenhang mit Fig. 1 darf darauf hingewiesen werden, dass das in Richtung des Pfeiles 30 in den Anschlussstutzen 21 eintretende Gas mittels eines in der Zeichnung nicht dargestellten äusseren Ventils im Atemtakt des Patienten steuerbar ist. Dieses Druckgas gelangt in den zentralen Ansaugkanal 25, der sich in seinem oberen Bereich zu einer schmalen Auslassdüse verjüngt. Durch die hohe Gasgeschwindigkeit in diesem Bereich wird aus den seitlichen Ansaugkanälen 24, 25 das zu zerstäubende Gut 26 angesaugt und gegen die der Mündungsöffnung des Düsenkopfes 23 gegenüberliegenden Prallflächen des Gasstromsteuers 27 geschleudert. An den schräg gestellten Prallflächen des Gasstromsteuers 27 werden die beiden seitlichen Aerosolfächer 38 und 39 erzeugt, deren Teilchenspektrum durch den nochmaligen intensiven Aufprall an der Innenwandung des benachbarten Prallschirmes 31 zugunsten eines besser lungengängigen Aerosols verbessert wird.

Der Rand 32 am Prallschirm 31 und der Rand 33 am Einsatz 20 wirken daher in der beschriebenen Weise als zusätzliche Filter zur Verbesserung des Teilchenspektrums zusammen. Zu grosse Teilchen werden aus dem Aerosol ausgeschieden und fliessen zum Zerstäubungsgut 26 im Behälter 18 zurück. In gleicher Weise wirkt auch die Tropfkante 44 zur Verstärkung der Filterwirkung der Ränder 32 und 33 und scheidet grössere Teilchen aus dem Aerosolstrom aus, bevor dieser durch den Austrittsstutzen 29 den Inhalator verlässt. Um sicherzustellen, dass die von der Tropfkante 44 abgeleitete Flüssigkeit auch wieder zum Zerstäubungsgut 26 zurückfliessen kann, sind Öffnungen 45 im unteren Bereich des kegeligen Wandteils 35 der Haube 20 vorgesehen.

Obwohl die mit Kompressor betriebenen bekannten Inhaliergeräte gemäss DE-C 1 147 355 auch in der Vergangenheit im Vergleich mit getesteten Geräten ähnlicher Konstruktion anderer Hersteller schon relativ gut abgeschnitten haben (vgl. die Zeitschrift der Stiftung Warentest Juni 1983, S. 32 bis 37, insbesondere die Seite 36), konnte der für die Aerosol-Therapie besonders wichtige intrathorakale Aerosolanteil mit dem Inhalator der Erfindung trotz seiner gegenüber den üblichen Therapiegeräten geringeren Bauhöhe auf weit über 50% gesteigert werden. Dabei wurde die medizinische Prüfung nach dem gleichen in der genannten Zeitschrift beschriebenen Verfahren vorgenommen.

Die Herstellung des Inhalators in Spritzgusstechnik aus einem geeigneten Kunststoff gestattet auch eine billige Herstellung des Zerstäuberkopfes im Spritzgiessverfahren. Ein derartiger Kopf kann daher auch als Einwegkopf bzw. Wegwerfkopf verwendet werden, wobei die zeitraubende Reinigung beim Einsatz des Verneblers nacheinander bei verschiedenen Patienten oder bei unterschiedlicher Medikation entfällt. Selbstverständlich kann man den Zerstäuberkopf auch für den wiederholten Gebrauch reinigen und aufheben.

Ein bevorzugtes Anwendungsgebiet für den Inhalator bietet die stationäre oder ambulante Behandlung von Patienten mit einer sich beispielsweise täglich wiederholenden Aerosol-Therapie; in einem derartigen Fall wird der Zerstäuberkopf mit dem Sammelbehälter einfach an dem Handgriff nach der Therapie entnommen, mit einem Namensschild für die Identifikation versehen und bis zur nächsten Therapie an geeignetem Ort abgestellt.

## PATENTANSPRÜCHE

1. Inhalator zum Zerstäuben, Verteilen und Vermischen von flüssigen und pulverförmigen Stoffen in/mit Gas mittels eines Druckgasstromes zur Erzeugung eines Aerosols, welcher einen Zerstäuberkopf (1) mit einem zentralen Düsenkopf (23) und einem Anschlussstutzen (21) für die Druckgasleitung enthält sowie einen Aerosolaustrittsstutzen (29) zum Anschluss an ein Mundstück für den Patienten, dadurch gekennzeichnet, dass der Inhalator einen den Zerstäuberkopf umschliessenden zweischaligen Handgriff (2) aufweist, dessen beide Hälften am unteren Ende des Griffteils mittels eines Filmscharniers (43) miteinander verbunden sind und dass der Zerstäuberkopf eine etwa kugelige, den Kugelschalenhälften (5, 6) des Griffteils angepasste Konfiguration aufweist.

2. Inhalator nach Anspruch 1, dadurch gekennzeichnet, dass der Handgriff (2) zur Aufnahme des Zerstäuberkopfes (1) aus den Griffschalen (3, 4) gebildet ist, deren obere Enden in Form von Kugelschalenhälften (5, 6) zur Aufnahme des Zerstäuberkopfes (1) geformt sind und die in ihren Griffteilen Positionierungsstege (15) zur Positionierung der Rippen (40) am Boden des Behälters (18) des Zerstäuberkopfes (1) aufweisen.

3. Inhalator nach Anspruch 2, dadurch gekennzeichnet, dass die beiden Griffschalenhälften (3, 4) mittels in ihrem Innenraum angeformter Verriegelungsösen (11) und mit im gegenüberliegenden Schalenteil angeformten Verriegelungshaken (12) miteinander lösbar verbindbar sind und dass Öffnungen (13) der Verriegelungsösen (11) mit entsprechenden Vorsprüngen (14) der Verriegelungshaken (12) zusammenwirken.

4. Inhalator nach den Ansprüchen 1 oder 2, dadurch gekennzeichnet, dass zur Positionierung des Zerstäuberkopfes (1) im Handgriff (2) Ausnehmungen (7a, 7b) an dem dem Scharnier entgegengesetzten Ende des Handgriffs für den Kaminfortsatz (41) des Zuluftkamins (28) und weitere dem Anschlussstutzen (21) für das Druckgas zugeordnete Ausnehmungen (10a, 10b) vorgesehen sind.

5. Inhalator nach den Ansprüchen 1 bis 4, dadurch gekennzeichnet, dass die Griffschalen (3, 4) des Handgriffs (2) im Bereich ihrer miteinander in Eingriff stehenden Ränder entweder mit Randleisten (16) oder mit den Randleisten auf der gegenüberliegenden Griffschale zusammenwirkenden Aussparungen (17) versehen sind.

6. Inhalator nach Anspruch 1, dadurch gekennzeichnet, dass der Zerstäuberkopf (1) von einem Düsenkopf (23) mit Gasstromsteuer (27) und einem das Zerstäubungsgut (26) aufnehmenden Behälter (18) sowie einer Haube (19) mit Zuluftkamin (28) und einem dazwischen angeordneten sich nach innen erstreckenden Einsatz (20) gebildet ist.

7. Inhalator nach Anspruch 6, dadurch gekennzeichnet, dass der Zuluftkamin (28) koaxial angeordnet und mit der Haube (19) einteilig ausgebildet ist.

8. Inhalator nach Anspruch 7, dadurch gekennzeichnet, dass der Zuluftkamin (28) einen Prallschirm (31) mit einem zylindrischen Fortsatz (31a) trägt, der dem Innendurchmesser des Zuluftkamins (28) angepasst ist.

9. Inhalator nach Anspruch 8, dadurch gekennzeichnet, dass der Prallschirm (31) sich vom Zuluftkamin (28) nach abwärts und auswärts glockenförmig, kugelig oder parabolisch nach aussen erstreckt und den freien Ringraum im Zerstäuberkopf (1) für das Aufsteigen des Aerosols verkleinert.

10. Inhalator nach Anspruch 9, dadurch gekennzeichnet, dass der Rand (32) des Prallschirms (31) sich nach unten bis in die Ebene der Stirnfläche des Düsenkopfes (23) erstreckt.

11. Inhalator nach Anspruch 6, dadurch gekennzeichnet, dass der Einsatz (20) ein kegeliges Wandteil (35) aufweist, welche sich nach einwärts und aufwärts in die Haube (19) erstreckt, wobei die obere Kante (33) des Einsatzes (20) mit der äusseren Mantelfläche des Zuluftkamins (28) einen weiteren Ringraum begrenzt, durch den das Aerosol in den Austrittsstutzen geleitet wird.

12. Inhalator nach Anspruch 11, dadurch gekennzeichnet, dass an der Innenwandung der Haube (19) eine Tropfkante (44) angeformt ist, die mit dem Rand (32) am Prallschirm (31) und dem Rand (33) am Einsatz (20) so zusammenwirkt, dass an den so gebildeten Umlenkstellen grössere Tröpfchen aus dem Aerosolstrom abgeschieden und in das Zerstäubungsgut 26 im Behälter (18) rückgeführt werden.

13. Inhalator nach Anspruch 6, dadurch gekennzeichnet, dass am oberen Rand des Behälters (18) eine Ringschulter (37) angeformt ist, auf welcher sich der Einsatz (20) mittels eines Flansches (36) abstützt.

## Claims

1. Inhaler for atomizing, dispersing and mixing of fluid and powdery substances in/with gas by means of a pressurized gas flow for producing an aerosol, which comprises an atomization head (1) with a central nozzle head (23) and a connection passage (21) for pressurized gas supply and an aerosol outlet passage (29) for connection to a mouthpiece for the patient, characterised in that the inhaler has a hollow two part hand grip (2) embracing the atomization head, the two parts being connected together at the lower end of the grip portion by means of a film hinge (43) and in that the atomization head has a somewhat spherical configuration adapted to the hollow parts (5, 6) of the grip portion.

2. Inhaler according to claim 1 characterised in that the hand grip (2) for receiving the atomization head (1) is constructed of hollow grip parts (3, 4) whose upper ends are formed in the form of hollow spherical halves (5, 6) for receiving the atomization head (1) and which have the positioning flanges (15) in their grip portions positioning the ribs (40) on the base of the container (18) of the atomization head (1).

3. Inhaler according to claim 2 characterised in that the two hollow grip parts (3, 4) are releasibly connectable together by means of latching lugs (1) formed in their interior and with latching hooks (12) formed in the oppositely lying hollow part, and in that openings (13) of the latching lugs cooperate with corresponding projections (14) of the latching hooks (12).

4. Inhaler according to claim 1 or 2 characterised in that for positioning the atomization head (1) in the hand grip (2), recesses are provided on the end of the hand grip opposite the hinge for a duct extension (41) of the air inlet duct (28) and further recesses (10a, 10b) are provided for the connection passage (21) for the pressurized gas.

5. Inhaler according to any of claims 1 to 4 characterised in that the hollow grip parts (3, 4) of the hand grip (2) are provided in the region of their mutually engaged edges either with peripheral flanges (16) or with grooves (17) cooperating with the peripheral flanges on the oppositely lying hollow part.

6. Inhaler according to claim 1 characterised in that the atomization head (1) is formed from a nozzle head (23) with gas flow control (27) and a container (18) receiving the atomization material (26), and a cap (19) having an air supply duct (28) and an inwardly extending intermediate insert (20).

7. Inhaler according to claim 6 characterised in that the air supply duct (28) is coaxially arranged and constructed in one piece with the cap (19).

8. Inhaler according to claim 7 characterised in that the air supply duct (28) carries a deflector (31) having a cylindrical extension (31a) which is adapted to the inner diameter of the air supply duct (28).

9. Inhaler according to claim 8 characterised in that the deflector (31) extends downwardly and outwardly of the air supply duct (28) in a campanulate shape and outwardly in a spherical or parabolic shape and diminishes the free annular space in the atomization head for the ascent of the aerosol.

10. Inhaler according to claim 9 characterised in that the edge (32) of a deflector (31) extends downwardly to the plane of the front surface of the nozzle head (23).

11. Inhaler according to claim 6 characterised in that the insert (20) has a conical wall part (35) which extends inwardly and upwards into the cap (19), the upper edge (33) of the insert (20) delimiting a further annular space with the outer sleeve surface of the air supply duct (28) through which the aerosol is conducted into the outlet passage.

12. Inhaler according to claim 11 characterised in that a drip edge (44) is formed on the inner wall of the cap (19) which drip edge (44) cooperates with the edge (32) on the deflector (31) and the edge (33) on the insert (20) in such manner that at the thus-formed deflection position larger drops are separated from the aerosol flow and are returned into the atomization material (26) in the container (18).

13. Inhaler according to claim 6 characterised in that on the upper edge of the container (18) an annular shoulder (37) is formed on which the insert (20) abuts by means of a flange (36).

## Revendications

1. Inhalateur pour atomiser, répartir et mélanger des substances liquides et sous forme pulvérulente dans ou avec un gaz, au moyen d'un courant de gaz sous pression dans le but de produire un aérosol, inhalateur comprenant une tête d'atomiseur (1) à tête de buse (23) centrale et une tubulure de raccordement (21) pour la conduite de gaz sous pression, ainsi qu'une tubulure de sortie d'aérosol (29) destinée au raccordement à une pièce de bouche pour le patient, caractérisé en ce que l'inhalateur présente une poignée (2) à deux coquilles entourant la tête d'atomiseur, dont les deux moitiés sont reliées entre elles à l'extrémité inférieure de la partie de prise au moyen d'une charnière à film (43) et en ce que la tête d'atomiseur présente une configuration à peu près sphérique et adaptée aux moitiés en coquilles (5, 6) de la partie de prise.

2. Inhalateur selon la revendication 1, caractérisé en ce que la poignée (2) destinée à recueillir la tête d'atomiseur (1) est formée à partir des coquilles de prise (3, 4) dont les extrémités supérieures sont formées en moitiés de coquilles sphériques (5, 6) dans le but de recueillir la tête d'atomiseur (1) et présentent dans leurs parties de prise des nervures de positionnement (15) destinés à positionner les nervures (40) situées sur le fond du réservoir (18) de la tête d'atomiseur (1).

3. Inhalateur selon la revendication 2, caractérisé en ce que les deux moitiés de coquilles de prise (3, 4) sont susceptibles, d'être reliées entre elles de manière démontable à l'aide d'œillets de verrouillage (11) formés dans leur espace intérieur et par des crochets de verrouillage (12) formés dans la partie en coquille opposée et en ce que des orifices (13) des œillets de verrouillage (11) coopèrent avec des saillies (14) correspondantes des crochets de verrouillage (12).

4. Inhalateur selon l'une des revendications 1 ou 2, caractérisé en ce que, dans le but de réaliser un positionnement de la tête d'atomiseur (1) dans la poignée (2), des évidements (7a, 7b) sont prévus sur l'extrémité de la poignée opposée à la charnière, pour le prolongement de cheminée (41) de la cheminée d'amenée d'air (28) et que d'autres évidements (10a, 10b) associés à la tubulure de raccordement (21) du gaz sous pression sont prévus.

5. Inhalateur selon les revendications 1 à 4, caractérisé en ce que les coquilles de prise (3, 4) de la poignée (2) sont pourvues, dans la zone de leurs bords placés en engagement conjoint, soit de bandes de bordure (16), soit d'évidements (17) coopérant avec les bandes de bordures situées sur la coquille de prise opposée.

6. Inhalateur selon la revendication 1, caractérisé en ce que la tête d'atomiseur (1) est formée d'une tête de buse (23) à déflecteur de courant de gaz (27) et d'un réservoir (18) recevant le produit à atomiser (26) ainsi que d'un capot (19) muni d'une cheminée d'air (28) et d'un insert (20) disposé de manière intermédiaire et s'étendant vers l'intérieur.

7. Inhalateur selon la revendication 6, caractérisé en ce que la cheminée d'air (28) est disposée coaxialement et qu'elle est réalisée d'une seule pièce avec le capot (19).

8. Inhalateur selon la revendication 7, caractérisé en ce que la cheminée d'air (28) porte un écran d'impact (31) à prolongement cylindrique (31a) qui est ajusté au diamètre intérieur de la cheminée d'amenée d'air (28).

9. Inhalateur selon la revendication 8, caractérisé en ce que l'écran d'impact (31) s'étend, en partant de la cheminée d'amenée d'air (28), vers le bas et vers l'extérieur en forme de cloche, sphérique ou parabolique vers l'extérieur et rétrécit dans la tête d'atomiseur (1) l'espace annulaire libre pour la montée de l'aérosol.

10. Inhalateur selon l'une des revendications 9, caractérisé en ce que le bord (32) de l'écran d'impact (31) s'étend vers le bas jusque dans le plan de la face frontale de la tête de buse (23).

11. Inhalateur selon la revendication 6, caractérisé en ce que l'insert (20) présente une partie de paroi (35) conique qui s'étend dans le capot (19) vers l'intérieur et vers le haut, l'arête supérieure (33) de l'insert (20) délimitant avec l'enveloppe de la cheminée d'amenée d'air (28) un autre espace annulaire à travers lequel l'aérosol est dirigé dans la tubulure de sortie.

12. Inhalateur selon la revendication 11, caractérisé en ce qu'une arête d'égoutture (44) est formée sur la paroi intérieure du capot (19) et coopère avec le bord (32) situé sur l'écran d'impact (31) et le bord (33) situé sur l'insert (20) de façon que les plus grosses gouttelettes situées sur les points de déviation ainsi formés soient séparées de l'aérosol et retournées dans le produit à atomiser (26) situé dans le réservoir (18).

13. Inhalateur selon la revendication 6, caractérisé en ce qu'il est formé sur le bord supérieur du réservoir (18) un épaulement annulaire (37) sur lequel l'insert (20) s'appuie au moyen d'un rebord (36).

# FIG. 1

# FIG. 2